# EUROPEAN PATENT SPECIFICATION

(11) **EP 0 553 294 B1**
(45) Date of publication and mention of the grant of the patent: **23.12.1998**
(21) Application number: 92900114.7
(22) Date of filing: 15.10.1991
(51) Int. Cl.: A61K 38/21

(54) **THE MANUFACTURE OF COMPOSITIONS FOR THE TREATMENT OF CELL PROLIFERATION DISORDERS**
DIE HERSTELLUNG VON ZUSAMMENSETZUNGEN ZUR BEHANDLUNG VON KRANKHEITEN DER ZELLPROLIFERATION
L'OBTENTION DES COMPOSITIONS DESTINES AU TRAITEMENT DES AFFECTIONS DUES A LA PROLIFERATION CELLULAIRE

(30) Priority: 17.10.1990 US 599206; 15.10.1991 US 772922
(43) Date of publication of application: 04.08.1993
(73) Proprietor: AMGEN INC., Thousand Oaks California 91320 -1789 (US)
(72) Inventor: BLATT, Lawrence, M., Ventura, CA 93003 (US); TAYLOR, Milton, W., Bloomington, IN 47401 (US)
(74) Representative: Brown, John David
(86) International application number: US9107722
(87) International publication number: WO9206707

(56) References cited:
- US-A- 4 695 623
- US-A- 4 879 471
- JOURNAL OF INTERFERON RESEARCH, vol.9, no.1, February 1989 pages 97 - 114 E.N. FISH ET AL 'The role of three domains in the biological activity of human Interferon-alpha'
- ANTIMICROBIAL AGENTS AND CHEMOTHERAPY, vol.30, no.1, July 1986 pages 52 - 56 E. N. FISH ET AL 'Antiherpetic effects of a human alpha Interferon analog, IFN-alphaCon1, in hamsters'
- ANTIVIRAL RESEARCH, vol.SUPPL1, 1985 pages 191 - 197 E.N. FISH ET AL 'Efficacy of consensus interferon alpha against HSV-2 infections'
- THE NEW ENGLAND JOURNAL OF MEDICINE, vol.308, no.18, 5 May 1983 pages 1071 - 1076 S.E. KROWN ET AL 'Preliminary observations on the effect of recombinant Leukocyte A Interferon in homosexual men with Kaposi's sarcoma'
- BRITISH JOURNAL OF HAEMATOLOGY, vol.60, 1985 pages 759 - 763 C. P. WORMAN ET AL 'Interferon is effective in Hairy cell leukaemia'
- LEUKEMIA, vol.1, no.4, April 1987 pages 317 - 319 J.R. QUESADA ET AL 'Mid-observations on the efficacy of alpha-interferon in Hairy Cell Leukemia and status of the interferon system of patients in remission'
- PROCEEDINGS OF THE AMERICAN SOCIETY OF CLINICAL ONCOLOGY, no.3, March 1984 page 55 F.X. REAL ET AL 'Treatment of Kaposi's sarcoma (KS) with recombinant Leukocyte A interferon (rIFN-alphaA)'
- JOURNAL OF INTERFERON RESEARCH, vol.10, November 1990, SUPPL 1 page S120 O.N. OZES ET AL 'A comparison of human consensus Interferon with other interferons for anti-viral and anti-proliferative activity'
- Blut, Volume 53, issued 1986, T. MORITZ et al., The Effect of Interferons on Cellular Defferention," pages 361-370, see entire document.
- Pharmac. Ther., Volume 27, issued 1985, P.B. Fisher, "Effects of Interferon on Differentiation of Normal and Tumor Cells", pages 143-166, see tables 1 en 2.
- Nature, Volume 290, issued 05 March 1981, D.V. GOEDDEL, et al. "The Structure of eight distinct cloned human leukocyte interferon CDNAS", pages 20-26.

## Description

The present invention relates to the manufacture of a medicament for use in methods of treating cellular proliferation disorders using consensus human leukocyte interferon. The invention also relates to the manufacture of pharmaceutical compositions of consensus human leukocyte interferon that are suitable for the treatment of cellular proliferation disorders.

### Background of the Invention

Interferons are a subclass of cytokines that exhibit both antiviral and antiproliferative activity. On the basis of biochemical and immunological properties, human interferons are grouped into three classes: interferon-alpha (leukocyte), interferon-beta (fibroblast) and interferon-gamma (immune). Fourteen alpha interferons (grouped into subtypes A through H) having distinct amino acid sequences have been identified by isolating and sequencing DNA encoding these polypeptides. Alpha interferons have received considerable attention as potential therapeutic agents due to their antiviral and antitumor growth inhibition.

The purification of interferon from human leukocytes isolated from the buffy coat fraction of whole blood is described in U.S. Patent No. 4,503,035. Human leukocyte interferon prepared in this manner contains a mixture of different human leukocyte interferon amino acid sequences. The purified material has a specific activity of from 0.9 x 10⁸-4 x 10⁸ units/mg. of protein when assayed on the MDBK bovine cell line and from 2 x 10⁶-7.6 x 10⁸ units/mg. of protein when assayed on the Ag 1732 human cell line. The cytopathic effect inhibition assay used to determine interferon anti-viral activity is disclosed in U.S. Patent No. 4,241,174. The measured interferon activity was calibrated against a reference standard for human leukocyte interferon provided by the National Institutes of Health.

The construction of recombinant DNA plasmids containing sequences encoding at least part of human leukocyte interferon and the expression in E. coli of a polypeptide having immunological or biological activity of human leukocyte interferon is disclosed in U.S. Patent No. 4,530,901.

The construction of hybrid alpha-interferon genes containing combinations of different subtype sequences (e.g., A and D, A and B, and A and F) is disclosed in U.S. Patent Nos. 4,414,150, 4,456,748, and 4,678,751.

U.S. Patents Nos. 4,695,623 and 4,897,471 disclose novel human leukocyte interferon polypeptides having amino acid sequences which include common or predominant amino acids found at each position among naturally-occurring alpha interferon subtype polypeptides and are referred to as consensus human leukocyte interferon (IFN-con). The IFN-con amino acid sequences disclosed are designated IFN-con₁, IFN-con₂, and IFN-con₃. The preparation of manufactured genes encoding IFN-con and the expression of said genes in E. coli are also disclosed.

A purification of IFN-con₁ produced in E. coli is described in Klein et al. (*J. Chromatog.* 454, 205-215 (1988)). IFN-con₁ purified in this manner is reported to have a specific activity of 3 x 10⁹ units/mg. protein as measured in the cytopathic effect inhibition assay using the T98G human cell line (Fish et al. *J. Interferon Res*. 9, 97-114 (1989)). Purified IFN-con₁ comprises three isoforms as determined by isoelectric focusing which have been identified as methionyl IFN-con₁, des-methionyl IFN-con₁ and des-methionyl IFN-con₁ with its N-terminus blocked by an acetyl group. (Klein et al. *Arch*. *Biochem. Biophys*. 276, 531-537 (1990)).

Alpha-interferon is currently approved in the United States and other countries for the treatment of hairy cell leukemia, venereal warts, Kaposi's Sarcoma (a cancer commonly afflicting patients suffering from Acquired Immune Deficiency Syndrome (AIDS)), and chronic non-A, non-B hepatitis. Two variants of alpha interferon have received approval for therapeutic use: Interferon alfa-2a, marketed under the trade name ROFERON-A, and Interferon alfa-2b, marketed under the trade name INTRON-A. The amino acid sequences of ROFERON-A and INTRON-A differ at a single position but otherwise are identical to the amino acid sequence of alpha-interferon subtype 2 (subtype A).

In addition to the labeled indications, alpha-interferon is being used or evaluated alone or in conjunction with chemotherapeutic agents in a variety of other cellular proliferation disorders, including chronic myelogenous leukemia, multiple myeloma, superficial bladder cancer, skin cancers (basal cell carcinoma and malignant melanoma), renal cell carcinoma, ovarian cancer, low grade lymphocytic and cutaneous T cell lymphoma, and glioma. Alpha-interferon may be effective in combination with other chemotherapy agents for the treatment of solid tumors that arise from lung, colorectal and breast cancer (see Rosenberg et al. "Principles and Applications of Biologic Therapy" in *Cancer: Principles and Practices of Oncology*, 3rd ed., Devita et al., eds. pp. 301-547 (1989), Balmer *DICP*, *Ann Pharmacother* 24, 761-768 (1990)).

Alpha-interferons are known to affect a variety of cellular functions, including DNA replication and RNA and protein synthesis, in both normal and abnormal cells. Thus, cytotoxic effects of interferon are not restricted to tumor or virus infected cells but are also manifested in normal, healthy cells as well. As a result, undesirable side effects arise during interferon therapy, particularly when high doses are required. Administration of interferon can lead to myelosuppression resulting in reduced red blood cell, white blood cell and platelet levels. Higher doses of interferon commonly give rise to flu-like symptoms (e.g., fever, fatigue, headaches and chills), gastrointestinal disorders (e.g., anorexia, nausea and diarrhea), dizziness and coughing. It would be useful to reduce or eliminate the undesirable side effects of interferon therapy without diminishing the therapeutic benefits of such therapy.

Therefore, an object of this invention is the treatment of cell proliferation disorders such as hairy cell leukemia or Kaposi's Sarcoma with IFN-con wherein the associated undesirable side effects are diminished compared to currently practiced treatment regimens or eliminated entirely. Alternatively, an object of the invention is enhanced therapeutic benefit in the treatment of cell proliferation disorders with IFN-con compared to currently practiced regimens with no corresponding increase in the frequency or severity of undesirable side effects.

### Summary of the Invention

The invention encompasses the manufacture of a medicament for use in methods of treating a cell proliferation disorder involving administering to a mammal a therapeutically effective amount of consensus human leukocyte interferon (IFN-con). It is shown that IFN-con has higher antiproliferative activity than INTRON-A. Therefore, treatment of a cell proliferation disorder using IFN-con shows enhanced efficacy and safety compared to other currently practiced interferon treatments. In particular, the administration of a therapeutically effective amount of IFN-con results in more rapid or more extensive treatment of a cellular proliferative disorder compared to currently practiced methods, wherein no corresponding increase in the frequency or severity of associated undesirable side effects occurs. In addition, a therapeutically effective amount of IFN-con may be less than said amount of an interferon used in currently practiced regimens. As a result, a decreased dose of IFN-con gives the same therapeutic benefit as higher doses of other interferons but with a decrease or elimination of undesirable side effects associated with currently practiced interferon therapy.

IFN-con is effective in treating cell proliferation disorders frequently associated with cancer. Such disorders include, but are not limited to, hairy cell leukemia and Kaposi's Sarcoma. IFN-con may be used alone or in combination with other therapeutics for the treatment of cancer and other proliferative disorders. In a preferred embodiment, IFN-con is used in conjunction with a therapeutically effective amount of one or more factors that stimulate myeloid cell proliferation or differentiation, such as granulocyte colony stimulating factor (G-CSF), granulocyte/macrophage colony stimulating factor (GM-CSF), interleukin-1 (IL-1), interleukin-3 (IL-3), interleukin-6 (IL-6), erythropoietin, and stem cell factor (SCF).

IFN-con is a nonnaturally-occurring polypeptide having antiproliferative activity. Preferably, IFN-con is a polypeptide having the amino acid sequence of IFN-con₁, IFN-con₂, or IFN-con₃. Most preferably, IFN-con has the amino acid sequence of IFN-con₁.

The invention also relates to the manufacture of pharmaceutical compositions comprising a therapeutically effective amount of IFN-con along with suitable diluents, adjuvants, carriers, preservatives and/or solubilizers.

### Brief Description of the Drawings

Figures 1 through 7 show the antiproliferative activity of IFN-con₁ and INTRON-A, a comparative material, on Eskol, a hairy cell leukemic cell line, when interferons were added to an Eskol cell suspension at 0.1, 0.5, 1, 5, 10, 50 and 100 ngs/ml, respectively.
Figure 8 shows the first and current median MTDs achieved by Kaposi's Sarcoma patients treated with INTRON-A, IFN-Con₁, or IFN-Con₁ and r-metGCSF.

### Detailed Description of the Invention

As employed herein, consensus human leukocyte interferon (IFN-con) shall mean a nonnaturally-occurring polypeptide which predominantly includes those amino acid residues which are common to all naturally-occurring human leukocyte interferon subtype sequences and which includes, at one or more of those positions wherein there is no amino acid common to all subtypes, an amino acid which predominantly occurs at that position and in no event includes any amino acid residue which is not extant in that position in at least one naturally-occurring subtype. IFN-con encompasses but is not limited to the amino acid sequences designated IFN-con₁, IFN-con₂ and IFN-con₃ which are disclosed in commonly owned U.S. Patents 4,695,623 and 4,897,471. DNA sequences encoding IFN-con are synthesized as described in the above-mentioned patents.

IFN-con polypeptides are the products of expression of manufactured DNA sequences transformed or transfected into bacterial hosts, especially E. coli. That is, IFN-con is recombinant IFN-con. IFN-con produced in E. coli is purified by procedures known to those skilled in the art and generally described in Klein et al., supra (1988) for IFN-con₁. Purified IFN-con may comprise a mixture of isoforms, e.g., purified IFN-con₁ comprises a mixture of methionyl IFN-con₁, des-methionyl IFN-con₁ and des-methionyl IFN-con₁ with a blocked N-terminus (Klein et al., supra (1990)). Alternatively, IFN-con may comprise a specific, isolated isoform. Isoforms of IFN-con are separated from each other by techniques such as isoelectric focusing which are known to those skilled in the art.

The subject invention provides for a method of treating a cell proliferation disorder involving administering a therapeutically effective amount of IFN-con. A preferred embodiment of the invention is a method of treatment involving administering a therapeutically effective amount of IFN-con₁, IFN-con₂, or IFN-con₃. Most preferably, a therapeutically effective amount of IFN-con₁ is administered.

IFN-con is useful for treatment of a variety of cell proliferation disorders, particularly various cancers. These disorders include, but are not limited to, hairy cell leukemia, Kaposi's Sarcoma, chronic myelogenous leukemia, multiple myeloma, superficial bladder cancer, skin cancer (basal cell carcinoma and malignant melanoma), renal cell carcinoma, ovarian cancer, low grade lymphocytic and cutaneous T cell lymphoma, and glioma.

IFN-con is used alone or in combination with other therapeutics for the treatment of cancer and other proliferative disorders. IFN-con is administered in conjunction with a therapeutically effective amount of one or more chemotherapy agents such as busulfan, 5-fluorouracil (5-FU), zidovudine (AZT), leucovorin, melphalan, prednisone, cyclophosphamide, dacarbazine, cisplatin, and dipyridamole. IFN-con may also be given in conjunction with cytokines such as interleukin-2 (IL-2).

A therapeutically effective amount of IFN-con may be administered in combination with a therapeutically effective amount of one or more factors that stimulate myeloid differentiation so as to overcome the effects of myelosuppression observed during interferon treatments. Such agents include, but are not limited to, G-CSF, GM-CSF, IL-1, IL-3, IL-6, erythropoietin and SCF. Stem cell factor (SCF) stimulates the proliferation of early hematopoietic progenitor cells and has been described in U.S. Serial No. 573,616, the disclosure of which is hereby incorporated by reference.

In the working examples provided, it is shown that IFN-con₁ is an effective antiproliferative agent against hairy cell leukemia and AIDS-associated Kaposi's Sarcoma.

The anti-proliferative activity of IFN-con₁ and INTRON-A assayed on Eskol cells, a hairy cell leukemic cell line, is shown in Example 1. It is shown that IFN-con₁ has greater anti-proliferative activity than INTRON-A over a wide range of concentrations. Similar results were obtained when IFN-con₁ was compared to INTRON-A. These results indicate that IFN-con₁ has greater therapeutic efficacy when administered at the same concentrations as INTRON-A. Alternatively, lower concentrations of IFN-con₁ are required to demonstrate therapeutic efficacy equivalent to that of INTRON-A.

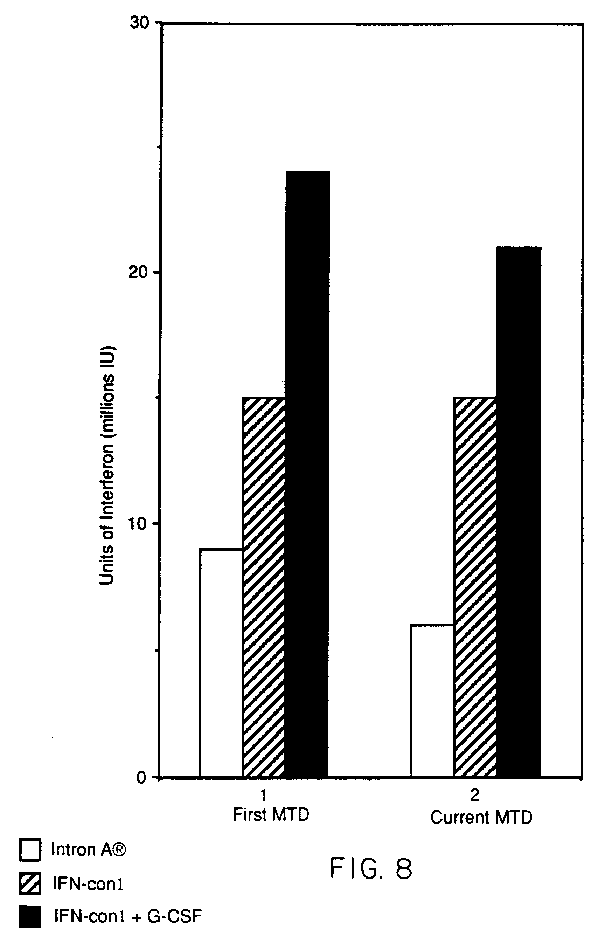

Example 2 describes a comparative study of IFN-Con₁ and INTRON-A in the treatment of AIDS-associated Kaposi's Sarcoma. It was shown that patients receiving IFN-Con₁ achieved higher unit doses than those patients receiving INTRON-A. In addition, patients receiving both and IFN-Con₁ and GCSF achieved higher doses of IFN-Con₁ than those patients receiving IFN-Con₁ alone (see Figure 8). In this study, all patients received AZT as part of their treatment of HIV infection. AZT administered alone is not effective on Kaposi's Sarcoma.

IFN-Con₁ was demonstrated to be safer than INTRON-A as judged by the reduced frequency of Grade 3 toxicity when IFN-Con₁ was administered. Treatment with IFN-Con₁ showed a reduced incidence of neutropenia and liver dysfunction compared to INTRON-A treatment while treatment with IFN-Con₁ and r-metGCSF completely eliminated Grade 3 toxicity (see Table 2).

Also provided for are pharmaceutical compositions comprising a therapeutically effective amount of IFN-con together with pharmaceutically acceptable carriers, adjuvants, diluents, preservatives and/or solubilizers. Pharmaceutical compositions of IFN-con include diluents of various buffers (e.g., Tris-HCl, acetate, phosphate) having a range of pH and ionic strength, carriers (e.g., human serum albumin), solubilizers (e.g., tween, polysorbate), and preservatives (e.g., thimerosol, benzyl alcohol). In general, components of pharmaceutical compositions can be selected from among those commonly employed with interferons and other antiproliferative agents and which are known to those skilled in the art. A pharmaceutical composition of IFN-con is supplied as an injectable solution or as a lyophilized powder which is reconstituted in an appropriate diluent prior to injection.

A therapeutically effective amount of IFN-con can be determined by one skilled in the art taking into account such variables as the half-life of IFN-con preparations, route of administration and the cell proliferation disorder being tested. In general, a therapeutically effective amount of IFN-con for the treatment of a cell proliferation disorder will be in the range of 2 x 10⁶ to 60 x 10⁶ units per patient administered several times per week. Doses in the lower part of the range are effective in the treatment of hairy cell leukemia while doses in the higher part of the range are suitable for the treatment of Kaposi's Sarcoma. Therapeutically effective amounts of IFN-con will preferably result in tumor remission of 20-80% depending upon the specific tumor type for a period of at least six months.

The route of administration will preferably be by injection into the blood of a mammal where the injection may be intravenous, intramuscular, subcutaneous or intralesional. The suitability of a given pharmaceutical composition for a given route of administration will be apparent to one skilled in the art.

The following examples are offered to more fully illustrate the invention but are not to be construed as limiting the scope thereof.

### EXAMPLE 1

### Anti-proliferative Activity of IFN-con₁ and INTRON-A

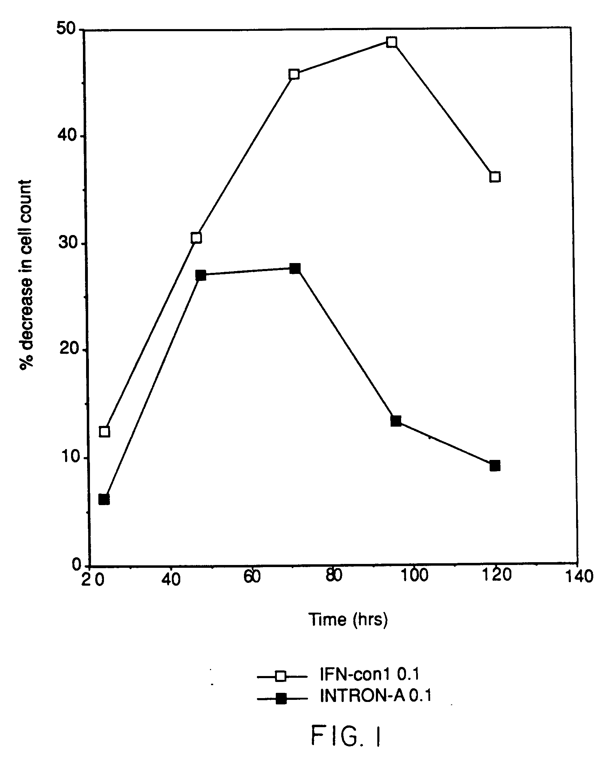

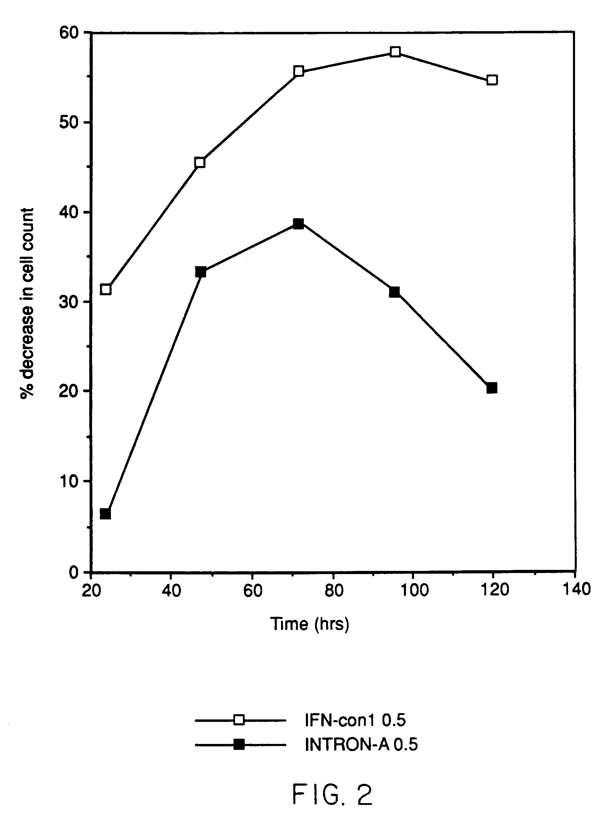

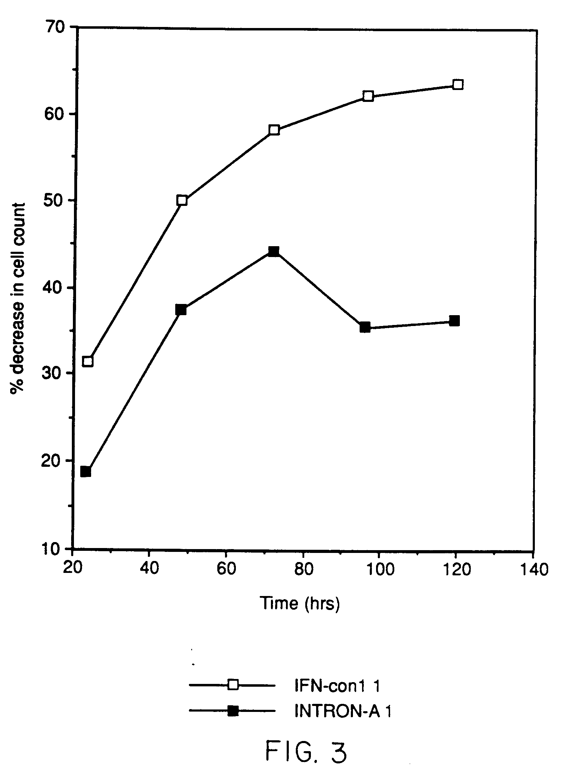

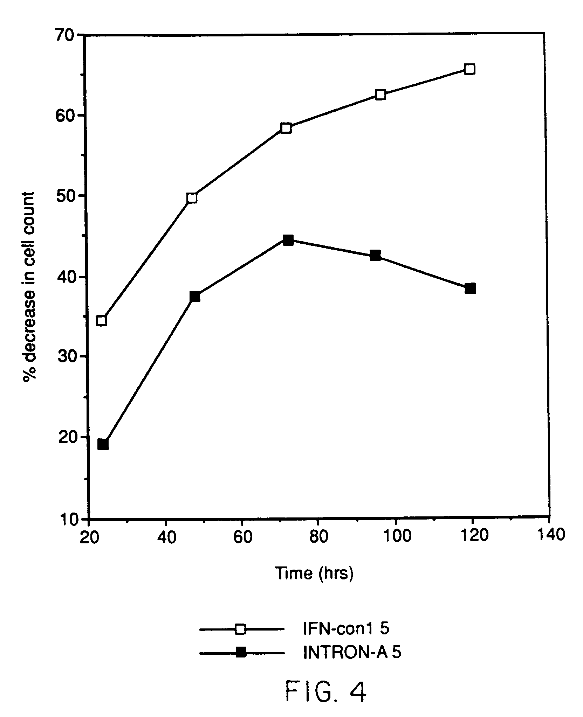

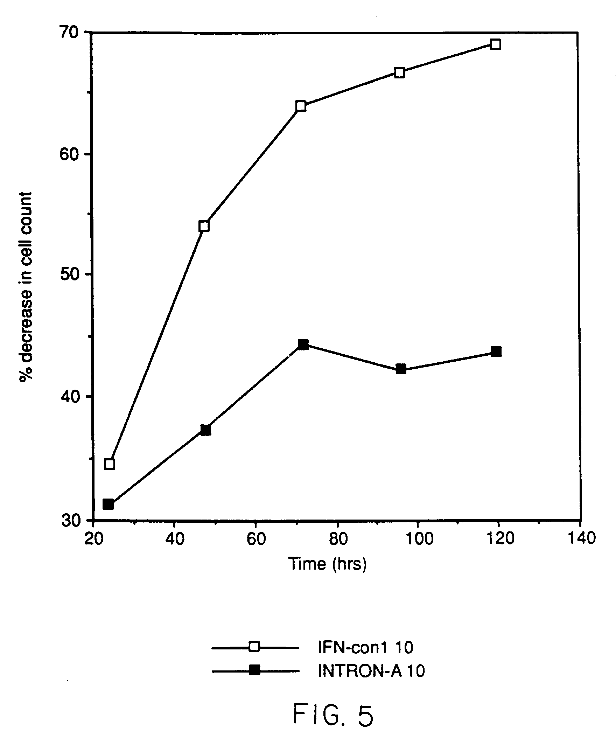

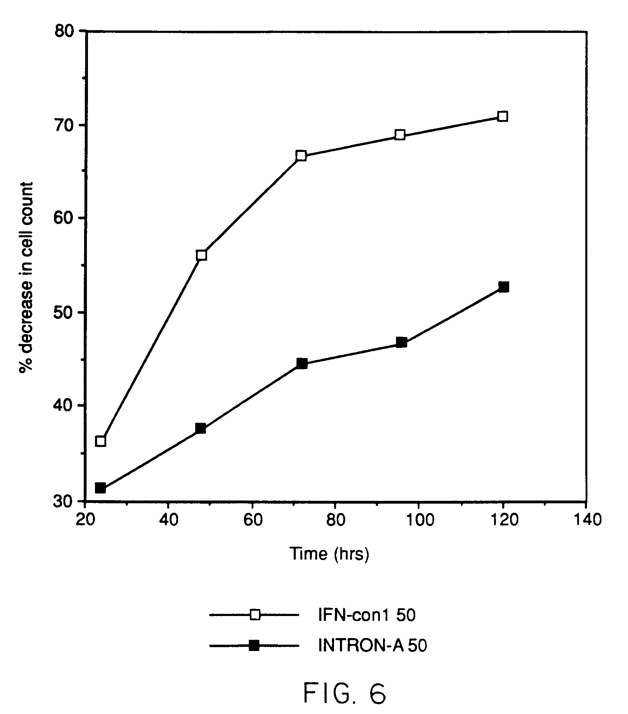

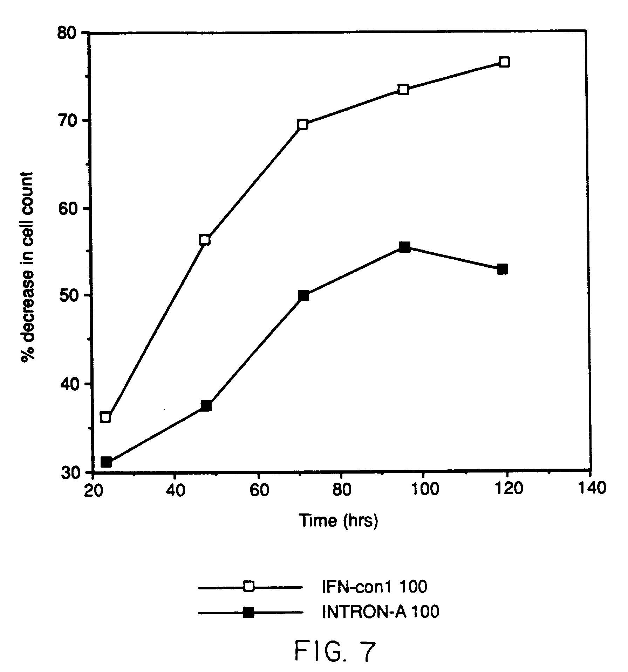

The anti-proliferative activity of IFN-con₁ and INTRON-A was tested on the Eskol cell line, a hairy cell leukemic cell line isolated by Dr. E. Srour at the Indiana University Medical School. Three ml cultures of Eskol cells were incubated in RPMI medium (Gibco) at 37°C in 5% CO₂ containing 10% fetal calf serum for 12 hours at 1 x 10⁵ cells/ml. IFN-con₁ or INTRON-A (Interferon alfa 2b; Schering Corp.) was added to a final protein concentration of 0.1 to 100 ngs/ml in 100 µl of media. The protein concentration of IFN-con₁ was determined by a Bradford protein assay (Bradford, *Anal. Biochem*. 72, 248-254 (1976)) while the concentration of INTRON-A was calculated from the specific activity (2 x 10⁸ International units/mg protein) and unit concentration supplied by the manufacturer. The number of viable cells was determined at 24 hour intervals by exclusion of trypan blue (Sigma). 100 µl of IFN-con₁ or INTRON-A were added to the indicated final concentration at 24 hour intervals. Viable cell counts were an average of four independent experiments with each experiment having duplicate samples. Variation in cell counts ranged from about 5% at 24 to 48 hours to about 2% at longer time points. The results shown in Figures 1-7 are ratios of viable cell counts in the presence or absence of interferon at various times expressed as percentages.

The viable cell count was confirmed by measuring the incorporation of ³H-thymidine into Eskol cells incubated in the presence of IFN-con₁ or INTRON-A. After the 120 hour incubation period, a 200 µl cell suspension was withdrawn and incubated at 37°C for three hours in the presence of 5 µCi/ml ³H-thymidine (Amersham). The cells were harvested using a Cambridge cell harvester (Cambridge Technology), washed seven times with distilled water and twice with 95% ethanol and the amount of ³H-thymidine incorporated was determined by liquid scintillation counting. The observed uptake of ³H-thymidine by Eskol cells incubated for 120 hours in the presence of IFN-con₁ or INTRON-A was proportional to the cell viability count.

### EXAMPLE 2

### Safety, Tolerance and Efficacy of IFN-con₁ Administered to Patients having Kaposi's Sarcoma (KS)

A randomized, open-label study to evaluate the safety and tolerance and to define the maximum tolerated dose (MTD) of IFN-con₁ and INTRON-A was undertaken. IFN-con₁ and INTRON-A were each administered in combination with zidovudine (AZT) to patients with AIDS-associated KS. In addition, the safety, tolerance and MTD of IFN-con₁ was determined when administered in conjunction with AZT and E. coli produced recombinant granulocyte colony stimulating factor having a methionine residue at the amino terminal end of the polypeptide (r-metGCSF). The three treatment groups in the study were:
1. INTRON-A and AZT
2. IFN-con₁ and AZT
3. IFN-con₁, AZT and r-metGCSF.
At least 12 evaluable patients are included in each treatment group.

### A. Product Description

IFN-con₁ was produced in E. coli using methods described in U.S. Patent Nos. 4,695,623 and 4,897,471. IFN-con₁ was purified by procedures generally described in Klein et al., supra (1988). For subcutaneous administration in the current study, IFN-con₁ was supplied as a sterile protein solution in sodium phosphate buffer. If required, dilution was made into sterile saline.

Zidovudine (AZT) was purchased from Burroughs-Wellcome Co. and used as directed on the package insert.

INTRON-A was purchased from Schering Corp. as a sterile, lyophilized formulation which was resuspended in diluent as directed on the package insert.

r-metGCSF was produced in E. coli using methods generally described in U.S. Patent No. 4,810,643, the disclosure of which is herein incorporated by reference. r-metGCSF was prepared as a sterile protein solution in 10 mM sodium acetate, 5% mannitol and 0.004% Tween 80 at pH 4.0 at a concentration of 0.3 mg/ml. If required, dilution was made into sterile 5% glucose in water (D₅W).

### B. Dosage and Schedules

AZT. AZT was administered to all patients at a fixed dose of 100 mg. orally every four hours while the patient is awake for a total of five doses, or 500 mg, daily.

r-metGCSF. For those patients randomized to the treatment group including r-metGCSF, doses of r-metGCSF were 1 µg/kg body weight per day, administered subcutaneously as a single bolus injection. If necessary, this dosage was increased in increments of 1 µg/kg/day (not to exceed 6 µg/kg/day) or decreased in decrements of 0.5 µg/kg/day or less, as appropriate, in order to achieve the absolute neutrophil count (ANC) target range of 5,000 to 15,000/mm³.

Interferon. Patients received either IFN-con₁ or INTRON-A according to a dose escalation scheme. Dosage was based upon equal units of either interferon. However, because the specific activities of the two interferons are different (2 x 10⁸ IU/mg for INTRON-A and at least 1 x 10⁹ IU/mg for IFN-con₁ as determined by the antiviral cytopathic assay described in U.S. Patent No. 4,695,623), the amount of protein by weight (in mg) at any given dose will be different for INTRON-A and IFN-con₁. The dose escalation scheme used is shown below in Table 1. The dose in mg of protein corresponding to each dose level in IUs is also shown in Table 1 for each interferon.

**TABLE 1**

| Dose Escalation Schedule for INTRON-A and IFN-Con₁ | | | |
|---|---|---|---|
| Dose Level | Dose x 10⁶ IU | Dose in mg Protein | |
| | | INTRON-A | IFN-Con₁ |
| 1 | 3 | 0.015 | 0.003 |
| 2 | 9 | 0.045 | 0.009 |
| 3 | 12 | 0.060 | 0.012 |
| 4 | 15 | 0.075 | 0.015 |
| 5 | 18 | 0.090 | 0.018 |
| 6 | 21 | 0.105 | 0.021 |
| 7 | 24 | 0.120 | 0.024 |
| 8 | 27 | 0.135 | 0.027 |
| 9 | 30 | 0.150 | 0.030 |

Patients in each of the three treatment groups shown above were administered IFN-con₁ or INTRON-A starting at dose level 1 daily for one week before escalating to the next highest dose level. Dose escalation occurred on days 8, 15, 22, 29, 36, 43, 50 and 57. Escalation continued until each patient reached an MTD or the maximum daily dose of 30 x 10⁶ IUs of interferon was achieved. The MTD for an individual patient was defined as the dose level below that at which dose-limiting toxicity occurs. Toxicity was graded on a scale of 0 (no toxicity) to 4 (acute toxicity) using criteria established by the World Health Organization and described further in Miller et al. (Cancer 47, 210-211 (1981). Dose-limiting toxicity was defined as a Grade 3 or Grade 4 adverse event judged to be at least possibly related to interferon. Fever and chills lasting less than 24 hours, fatigue, headache, or Grade 2 or less toxicity were not used in defining the MTD unless they were determined to be intolerable to the individual patient.

At the completion of the escalation phase, patients were continued on maintenance therapy consisting of daily dosing at either the patient's MTD or at the maximum dose of 30 x 10⁶ IUs if that was achieved. Maintenance therapy was continued until disease progression or other criteria warranted removing the patient from the study.

During maintenance therapy, two interferon dose reductions as a result of toxicity were permitted. After two dose reductions, no further interferon dosing modifications were allowed and patients requiring further reductions were withdrawn from the program. An exception to this procedure was when the dose-limiting toxicity was neutropenia (ANC ≤ 1000/mm³ on two days of an approximately one week period). In this instance, the patient was allowed to remain in the study without further reduction in interferon dose, but r-metGCSF therapy was initiated at 1 µg/kg body weight per day, administered subcutaneously, to patients not receiving r-metGCSF. For patients already in the r-metGCSF treatment group, the dose of r-metGSCF administered was escalated to the next highest level (an increase of 1 µg/kg/day).

### C. Patient Selection

A total of 49 patients have been enrolled in the study. An individual is enrolled in the study only after meeting all inclusion and exclusion criteria. The significant criteria for inclusion are serologically documented HIV infection, histopathologically confirmed Kaposi's Sarcoma with measurable cutaneous or oral lesion(s), acceptable immune function (as measured by CD4 lymphocyte levels) and under AZT treatment for less than one year.

Among the reasons for the withdrawal of a patient from the study are a second occurrence of Grade 3 or greater toxicity during the dose escalation phase, a third occurrence of dose-limiting toxicity after the individual patient's MTD has been determined and the patient is on maintenance therapy, or a progression in KS.

### D. Determination of MTDs for IFN-con₁ and INTRON-A

Using the dose escalation scheme described above for weeks 1-9 of study, followed by maintenance therapy and dose reduction when appropriate, the first and current median MTDs of INTRON-A AND IFN-Con₁ for the three treatment groups were determined and are shown in Figure 8. Each group consists of 15 patients. Group I (INTRON-A and AZT) attained a first MTD during dose escalation of 9 x 10⁶ IUs and a current MTD of 6 x 10⁶ IUs; Group 2 (IFN-con₁ and AZT) attained first and a current MTDs of 15 x 10⁶ IUs; and Group 3 (IFN-con₁, r-metGCSF and AZT) attained first and current MTDs of 24 x 10⁶ IUs and 21 x 10⁶ IUs, respectively.

### E. Evaluation of safety of INTRON-A and IFN-Con₁ treatment

The safety of INTRON-A and IFN-Con₁ treatment was determined by the severity of adverse effects that required interferon dose reduction. The results are summarized in Table 2.

**TABLE 2**

| Toxicities Prompting Dose Reductions In Three Treatment Groups | | | |
|---|---|---|---|
| | Frequency of Occurrence (%) | | |
| | INTRON-A | IFN-Con₁* | IFN-Con₁ and r-metGCSF* |
| Grade 2 Intolerance (Flu-like syndrome) | 20 | 70 | 65 |
| Grade 3 Neutropenia | 40 | 10 | 0 |
| Grade 3 Liver function tests | 30 | 10 | 0 |

| | | | |
|---|---|---|---|
| *Percentages for IFN-Con₁ and IFN-Con₁ and r-metGCSF treatment groups do not add up to 100% because some patients in these groups reached the maximum dose of 30 x 10⁶ IU with no adverse effects. | | | |
| Since the study was initiated, no patients have been withdrawn as a result of toxicity clearly resulting from the administration of INTRON-A or IFN-Con₁. | | | |

### F. Determination of efficacy of IFN-con₁ and INTRON-A treatment

Antitumor response. Antitumor responses were assessed after four months of treatment using the AIDS Clinical Trials Group (ACTG) Oncology Committee's standard response criteria (Krown et al. *J. Clin. Oncol*. 7, 1201-1207 (1989)).

Immune functions. CD4 lymphocyte counts are taken every month for six months during the study to evaluate patients' immune response to HIV infection.

In all three treatment groups, the Kaposi's Sarcoma lesion responses and CD4 lymphocyte levels were equivalent.

## Claims

1. Use of consensus human leukocyte interferon for the manufacture of a medicament for treatment of a cell proliferation disorder in a mammal, in which one or more side effects generally associated with alpha interferon administration is reduced.

2. A use according to Claim 1, wherein the cell proliferation disorder is hairy cell leukemia.

3. A use according to Claim 1, wherein the cell proliferation disorder is Kaposi's Sarcoma.

4. A use according to Claim 1, 2 or 3, wherein the consensus human leukocyte interferon is selected from IFN-con₁, IFN-con₂, and IFN-con₃.

5. A use according Claim 4, wherein the consensus human leukocyte interferon is IFN-con₁.

6. A use according to any one of the preceding claims, wherein the consensus human leukocyte interferon is the product of procaryotic expression of an exogenous DNA sequence.

7. A use according to any one of the preceding claims, wherein the medicament is for administration by intravenous, intramuscular, subcutaneous or intralesional routes.

8. A use according to any of the preceding claims, wherein the medicament is for administration in an amount of from 2 x 10⁶ to 60 x 10⁶ units of consensus human leukocyte interferon per patient.

9. A use according to any one of the preceding claims, wherein the mammal is a human.

10. A use according to any one of the preceding claims, wherein the medicament also comprises a therapeutically effective amount of a chemotherapeutic agent.

11. A use according to any one of Claims 1 to 9, wherein the medicament also comprises a therapeutically effective amount of G-CSF, GM-CSF, IL-1, IL-3 or SCF.

## Patentansprüche

1. Verwendung von Consensus-Humanleukozyteninteron zur Herstellung eines Arzneimittels zur Behandlung einer Zellproliferationserkrankung in einem Säuger, bei der eine oder mehrere Nebenwirkungen, die im allgemeinen mit der Verabreichung von alpha-Interferon assoziiert sind, verringert sind.

2. Eine Verwendung nach Anspruch 1, wobei die Zellproliferationserkrankung Haarzell-Leukämie ist.

3. Eine Verwendung nach Anspruch 1, wobei die Zellproliferationserkrankung Kaposi-Sarkom ist.

4. Eine Verwendung nach Anspruch 1, 2 oder 3, wobei dar Consensus-Humanleukozyteninterferon ausgewählt wird aus IFN-con₁, IFN-con₂ und IFN-con₃.

5. Eine Verwendung nach Anspruch 4, wobei das Consensus-Humanleukozyteninterferon IFN-con₁ ist.

6. Eine Verwendung nach einem der vorangehenden Ansprüche, wobei das Consensus-Humanleukozyteninterferon das Produkt prokaryontischer Expression einer exogenen DNA-Sequenz ist.

7. Eine Verwendung nach einem der vorangehenden Ansprüche, wobei das Arzneimittel zur Verabreichung durch intravenöse, intramuskuläre, subkutane oder intraläsionale Wege gedacht ist.

8. Eine Verwendung nach einem der vorangehenden Ansprüche, wobei das Arzneimittel zur Verabreichung in einer Menge von 3 x 10⁶ bis 60 x 10⁶ Einheiten Cosensus-Humanleukozyteninterferon pro Patient gedacht ist.

9. Eine Verwendung nach einem der vorangehenden Ansprüche, wobei der Säuger ein Mensch ist.

10. Eine Verwendung nach einem der vorangebenden Ansprüche, wobei das Arzneimittel auch eine therapeutisch wirksame Menge eines chemotherapeutischen Mittels umfaßt.

11. Eine Verwendung nach einem der Ansprüche 1 bis 9, wobei das Arzneimittel auch eine therapeutisch wirksame Menge G-CSF, GM-CSF, IL-1, IL-3 oder SCF umfaßt.

## Revendications

1. Utilisation de l'interféron de leucocytes humains consensus pour la fabrication d'un médicament pour le traitement d'un trouble de prolifération cellulaire chez un mammifère, dans laquelle un ou plusieurs effets secondaires généralement associés à l'administration de l'interféron alpha sont réduits.

2. Utilisation selon la revendication 1, dans laquelle le trouble de prolifération cellulaire est la leucémie de cellules velues.

3. Utilisation selon la revendication 1, dans laquelle le trouble de prolifération cellulaire est le sarcome de Kaposi.

4. Utilisation selon la revendication 1, 2 ou 3, dans laquelle l'interféron de leucocytes humains consensus est choisi à partir de IFN-con₁, IFN-con₂, et IFN-con₃.

5. Utilisation selon la revendication 4, dans laquelle l'interféron de leucocytes humains consensus est IFN-con₁.

6. Utilisation selon l'une quelconque des revendications précédentes, dans laquelle l'interféron de leucocytes humains concensus est le produit de l'expression procaryote d'une séquence d'ADN exogène.

7. Utilisation selon l'une quelconque des revendications précédentes, dans laquelle le médicament est destiné à être administré par des voies intraveineuse, intramusculaire, sous-cutanée ou à travers des lésions.

8. Utilisation selon l'une quelconque des revendications précédentes, dans laquelle le médicament est destiné à être administré en une quantité de 2 x 10⁶ à 60 x 10⁶ unités d'interféron de leucocytes humains consensus par patient.

9. Utilisation selon l'une quelconque des revendications précédentes, dans laquelle le mammifère est un être humain.

10. Utilisation selon l'une quelconque des revendications précédentes, dans laquelle le médicament comprend également une quantité thérapeutiquement efficace d'un agent chimiothérapeutique.

11. Utilisation selon l'une quelconque des revendications 1 à 9, dans laquelle le médicament comprend également une quantité thérapeutiquement efficace de G-CSF, GM-CSF, IL-1, IL-3 ou SCF.
